# EUROPEAN PATENT APPLICATION

(11) **EP 2 460 814 A1**
(43) Date of publication of application: **06.06.2012**
(21) Application number: 10804358.9
(22) Date of filing: 26.07.2010
(51) Int. Cl.: C07K 5/062, A61K 38/00, A61K 38/55, A61P 35/00, A61P 43/00

(54) **PEPTIDIC COMPOUND AND USE OF SAME**

(30) Priority: 30.07.2009 JP 2009178168
(71) Applicant: Keio University, Tokyo 108-8345 (JP)
(72) Inventor: SUENAGA, Kiyotake, Yokohama-shi Kanagawa 223-8522 (JP); TERUYA, Toshiaki, Yokohama-shi Kanagawa 223-8522 (JP); SASAKI, Hiroaki, Yokohama-shi Kanagawa 223-8522 (JP)
(74) Representative: HOFFMANN EITLE
(86) International application number: PCT/JP2010/062514
(87) International publication number: WO 2011/013612

(57) **Abstract**

An object of the present invention is to provide a novel compound having an antitumor/anticancer effect. Specifically, the present invention provides a compound represented by Formula (I), a derivative thereof or a salt of the same. In Formula (I), R¹ represents an alkyl group, an alkenyl group, a cycloalkyl group, an amino group, a monoalkylamino group, a dialkylamino group, an aryl group, a heteroaryl group, an aralkyl group or an alkoxy group; R² represents a substituted or unsubstituted amino group or a substituted or unsubstituted nitrogen-containing heterocyclic group; and R³ represents hydrogen, halogen, alkyl, methoxy, cyano, trifluoromethyl, acetylamino or amino. In Formula (I), N-Me-Ala may be substituted with N-Me-Gly, N-Me-Ser, N-Me-Thr, Ala, Gly, Ser or Thr; N-Me-Phe may be substituted with Phe, Tyr or N-Me-Tyr; and D-Leu may be substituted with L-Leu.

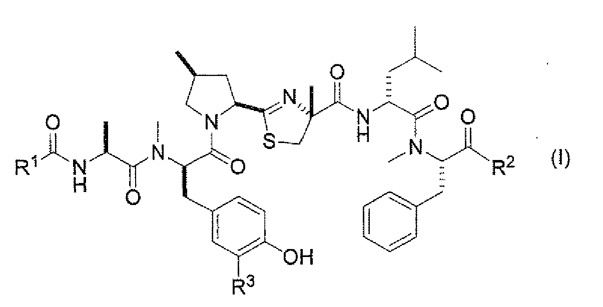

## Description

### Technical Field

The present invention relates to a peptidic compound or a derivative thereof, and the use of the same. More specifically, the present invention relates to anticancer drugs comprising the peptidic compound and MAPK phosphorylation inhibitors, in particular, ERK-selective phosphorylation inhibitors.

### Background Art

About half of the drugs that are currently in clinical use are of natural origin (Non-patent Literature 1), which indicates that natural products play a highly significant role in the drug discovery and development process (Non-patent Literature 2). Natural products, especially those from terrestrial plants and microbes, have long been a traditional source of drug molecules. Indeed, pharmacologically active compounds from plants and microbes play important roles in investigational new drugs. Much attention has recently been given to physiologically active substances from marine organisms (Non-patent Literature 3). In particular, cyanobacteria produce large amounts of bioactive secondary metabolites, and are considered to be a source of potential pharmaceuticals. For example, TZT-1027, a synthetic analog of dolastatin 10, is currently being evaluated in phase I clinical trials in Japan, Europe and the United States (Non-patent Literature 4).

### Citation List

### [Non-patent Literature]

NPL 1: Newman, D. J.; Cragg, G. M. J. Nat. Prod. 2007, 70, 461-477.
NPL 2: Koehn, F. E.; Carter, G. T. Nat. Rev. Drug Discovery 2005, 4, 206-220
NPL 3: Molinski, T. F.; Dalisay, D. S.; Lievens, S. L.; Saludes, J.P.; Nat. Rev. Drug Discovery 2009, 8, 69-85.
NPL 4: Horti, J.; Juhasz, E.; Monostori, Z.; Maeda, K.; Eckhardt, S.; Bodrogi, I. Cancer Chemother. Pharmacol. 2008, 62, 173-180.

### Summary of Invention

### Technical Problem

An object of the present invention is to provide a novel compound having an antitumor/anticancer effect.

### Solution to Problem

The present inventors searched for a novel physiologically active substance from the cyanobacterium of the genus *Lynghya* sp. collected in Okinawa Prefecture, using its antiproliferative activity against HeLa S₃ cells as an indicator; and succeeded in isolating Bisebromoamide (1), which exhibits antiproliferative activity against various cancers. The present inventors conducted research on the derivatives of the compound to complete the present invention.

The present invention provides the following compounds and anticancer drugs.
Item 1. A compound represented by Formula (I) below, a derivative thereof, or a salt thereof:

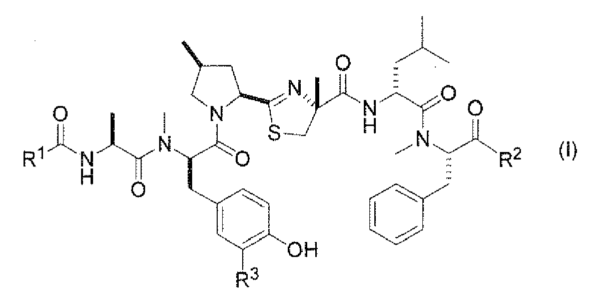

wherein R¹ represents an alkyl group, an alkenyl group, a cycloalkyl group, an amino group, a monoalkylamino group, a dialkylamino group, an aryl group, a heteroaryl group, an aralkyl group or an alkoxy group;

R² represents a substituted or unsubstituted amino group, or a substituted or unsubstituted nitrogen-containing heterocyclic group;

R³ represents hydrogen, halogen, alkyl, methoxy, cyano, trifluoromethyl, acetylamino or amino;

provided that Ala in Formula (I) may be substituted with N-Me-Gly, N-Me-Ser, N-Me-Thr, N-Me-Ala, Gly, Ser or Thr;

N-Me-Phe may be substituted with Phe, Tyr or N-Me-Tyr; and

D-Leu may be substituted with L-Leu.
Item 2. The compound of Item 1, a derivative thereof, or a salt thereof, wherein R² represents NR⁴R⁵ (wherein R⁴ and R⁵ are the same or different, and each represents a hydrogen atom, an alkyl group, or a cycloalkyl group),

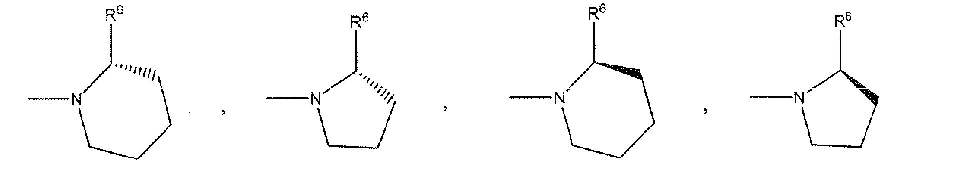

wherein R⁶ represents R⁷-CO- or a hydrazide thereof, or R⁷-CH(OR⁸)-(wherein R⁷ represents an alkyl group, an aryl group, or an aralkyl group; and R⁸ represents a hydrogen atom, an alkyl group, an aryl group, an aralkyl group, or an acyl group).
Item 3. The compound of Item 1 represented by Formula 1 below, a derivative thereof, or a salt thereof:

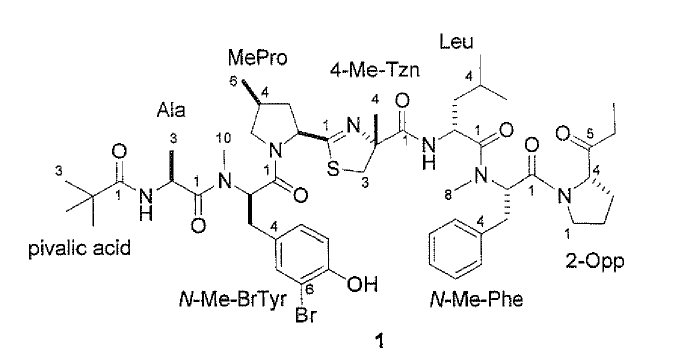

Item 4. An anticancer drug comprising the compound of any one of Items 1 to 3, a derivative thereof, or a salt thereof as an active ingredient.
Item 5. An ERK-selective phosphorylation inhibitor comprising the compound of any one of Items 1 to 3, a derivative thereof, or a salt thereof as an active ingredient.

### Advantageous Effects of Invention

The present invention provides a novel compound having an antitumor/anticancer effect.

Roberts P. J. and Der C. J.; Oncogene 2007, 26, 3291-3310 discloses that the activation of MAPK, and aberrant activation of phosphorylation of ERK are related to the proliferation of cancer.

The compound of the present invention exhibits a strong protein kinase inhibitory activity, and selectively inhibits phosphorylation of ERK. Due to its extremely high selectivity, the compound of the present invention is a potential anticancer drug effective for cancers with MAPK signaling pathways aberrantly activated.

### Brief Description of Drawings

Fig. 1 shows the scheme isolating Bisebromoamide (1) from cyanobacterium (*Lyngbya* sp.) collected in Okinawa.
Fig. 2 shows the levels of protein phosphorylation inhibitory activity of Bisebromoamide (1) obtained by the present invention. Fig. 2 indicates that Bisebromoamide (1) at a concentration of 0.1 to 10 µM selectively inhibited the phosphorylation of ERK without inhibiting the phosphorylation of signal transduction molecules other than ERK.
Fig. 3 shows the structural determination of Bisebromoamide (1).
Fig. 4 shows the measurement results of the growth inhibitory activity against various cancers.

### Description of Embodiments

The present invention provides a compound represented by Formula (I) below, a derivative thereof, or a salt thereof:

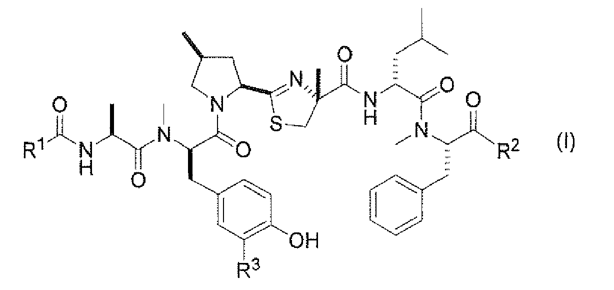

wherein R¹ represents an alkyl group, an alkenyl group, a cycloalkyl group, an amino group, a monoalkylamino group, a dialkylamino group, an aryl group, a heteroaryl group, an aralkyl group or an alkoxy group;

R² represents a substituted or unsubstituted amino group, or a substituted or unsubstituted nitrogen-containing heterocyclic group;

R³ represents hydrogen, halogen, alkyl, methoxy, cyano, trifluoromethyl, acetylamino or amino;

provided that Ala in Formula (I) may be substituted with N-Me-Gly, N-Me-Ser, N-Me-Thr, N-Me-Ala, Gly, Ser or Thr;

N-Me-Phe may be substituted with Phe, Tyr or N-Me-Tyr; and D-Leu may be substituted with L-Leu.

In the present specification, "halogen" means fluorine, chlorine, bromine and iodine; and fluorine, chlorine, and bromine are preferable.

Examples of "substituted or unsubstituted amino group" include an amino group, a monoalkylamino group, and a dialkylamino group.

Examples of heterocyclic moieties of the "substituted or unsubstituted nitrogen-containing heterocyclic group" include a pyrrolidinyl group, a piperidino group, a piperazinyl group, and like 5-membered or 6-membered nitrogen-containing heterocyclic groups. Examples of substituents include R⁷-CO- or a hydrazide thereof, or R⁷-CH(OR⁸)- (wherein R⁷ represents an alkyl group, an aryl group, or an aralkyl group; and R⁸ represents a hydrogen atom, an alkyl group, an aryl group, an aralkyl group, or an acyl group). Hereinafter, "alkyl," in a portion of a group, such as alkyl in alkylcarbonyl, is referred to as "alkyl moiety." Similarly, "aryl" in a portion of a group, such as aryl in arylcarbonyl, is referred to as "aryl moiety"; and "aralkyl" in a portion of a group, such as aralkyl in aralkylcarbonyl, is referred to as "aralkyl moiety."

The "alkyl group" or "alkyl moiety" may be linear, branched, or cyclic. Examples thereof include methyl, ethyl, n-propyl, isopropyl, n-butyl, isobutyl, tert-butyl, n-pentyl, isopentyl, hexyl, heptyl, octyl, nonyl, decyl, and like C₁-₁₀, preferably C₁₋₆ alkyl groups.

The "alkoxy group" may be linear, branched, or cyclic. Examples thereof include methoxy, ethoxy, n-propoxy, isopropoxy, n-butoxy, isobutoxy, tert-butoxy, n-pentoxy, isopentoxy, hexyloxy, and like C₁₋₆ alkoxy groups.

Specific examples of "cycloalkyl group" include cyclopropyl, cyclobutyl, cyclopentyl, cyclohexyl, and cycloheptyl.

The "alkenyl group" may be linear, branched, or cyclic, as long as it has at least one double bond. Examples thereof include vinyl, allyl, 1-propenyl, 2-methyl-2-propenyl, isopropenyl, 1-, 2- or 3-butenyl, 2-, 3- or 4-pentenyl, 2-methyl-2-butenyl, 3-methyl-2-butenyl, 5-hexenyl, 1-cyclopentenyl, 1-cyclohexenyl, and 3-methyl-3-butenyl, and like C₂₋₁₀, preferably C₂₋₆ alkenyl groups.

Examples of monoalkyl-substituted amino groups include methylamino, ethylamino, n-propylamino, isopropylamino, n-butylamino, isobutylamino, tert-butylamino, n-pentylamino, isopentylamino, hexylamino, and like C₁₋₆ alkyl-substituted amino groups.

Examples of dialkyl-substituted amino groups include dimethylamino, diethylamino, di-n-propylamino, diisopropylamino, di-n-butylamino, diisobutylamino, di-tert-butylamino, di-n-pentylamino, diisopentylamino, dihexylamino, and like amino groups disubstituted with a C₁₋₆ alkyl group.

The term "aryl group" or "aryl moiety" refers to a monocyclic or polycyclic group comprising a 5-membered or 6-membered aromatic hydrocarbon ring. Specific examples thereof include phenyl, naphthyl, fluorenyl, anthryl, biphenylyl, tetrahydronaphthyl, chromanyl, 2,3-dihydro-1,4-dioxanaphthalenyl, indanyl, and phenantolyl.

Examples of "aralkyl group" or "aralkyl moiety" include benzyl and phenethyl.

The "heteroaryl group" refers to a monocyclic or polycyclic group comprising a 5-membered or 6-membered aromatic ring containing 1 to 3 hetero atoms selected from N, O and S. In the case of a polycyclic group, at least one ring should be an aromatic ring. Specific examples thereof include furyl, thienyl, pyrrolyl, imidazolyl, pyrazolyl, oxazolyl, thiazolyl, isoxazolyl, isothiazolyl, pyridyl, pyrazinyl, pyrimidinyl, pyridazinyl, indolyl, quinolyl, isoquinolyl, benzo[b]thienyl, and benzimidazolyl.

The term "acylated hydroxy (i.e., in OR⁸, R⁸ is an acyl group)" refers to C₁₋₆ alkylcarbonyloxy, arylcarbonyloxy, or aryl-substituted C₁₋₆ alkylcarbonyloxy.

The term "etherified hydroxy (i.e., in OR, R is an alkyl, aralkyl, or aryl group)" refers to C₁₋₆ alkyloxy, aryloxy, or aryl-substituted C₁₋₄ alkyloxy.

Specific examples of C₁₋₆ alkylcarbonyloxy include methylcarbonyloxy, ethylcarbonyloxy, n-propylcarbonyloxy, isopropylcarbonyloxy, n-butylcarbonyloxy, isobutylcarbonyloxy, tert-butylcarbonyloxy, n-pentylcarbonyloxy, isopentylcarbonyloxy, and hexylcarbonyloxy.

Specific examples of arylcarbonyloxy include phenylcarbonyloxy, naphthylcarbonyloxy, fluorenylcarbonyloxy, anthrylcarbonyloxy, biphenylylcarbonyloxy, tetrahydronaphthylcarbonyloxy, chromanylcarbonyloxy, 2,3-dihydro-1,4-dioxanaphthalenylcarbonyloxy, indanylcarbonyloxy and phenanthrylcarbonyloxy.

Specific examples of aryl-substituted C₁₋₄ alkylcarbonyloxy include benzylcarbonyloxy, naphthylmethylcarbonyloxy, fluorenylmethylcarbonyloxy, anthrylmethylcarbonyloxy, biphenylylmethylcarbonyloxy, tetrahydronaphthylmethylcarbonyloxy, chromanylmethylcarbonyloxy, 2,3-diliydro-1,4-dioxanapthalenylmethylcarbonyloxy, indanylmethylcarbonyloxy, phenanthrylmethylcarbonyloxy, phenethylcarbonyloxy, naphthylethylcarbonyloxy, fluorenylethylcarbonyloxy, anthrylethylcarbonyloxy, biphenylylethylcarbonyloxy, tetrahydronaphthylethylcarbonyloxy, chromanylethylcarbonyloxy, 2,3-diliydro-1,4-dioxanapthalenylethylcarbonyloxy, indanylethylcarbonyloxy, and phenanthrylethylcarbonyloxy.

Specific examples of C₁₋₆ alkyloxy include methyloxy, ethyloxy, n-propyloxy, isopropyloxy, n-butyloxy, isobutyloxy, tert-butyloxy, n-pentyloxy, isopentyloxy, and hexyloxy.

Specific examples of aryloxy include phenyloxy, naphthyloxy, fluorenyloxy, anthryloxy, biphenylyloxy, tetrahydronaphthyloxy, chromanyloxy, 2,3-diliydro-1,4-dioxanapthalenyloxy, indanyloxy, and phenanthryloxy.

Specific examples of aryl-substituted C₁₋₄ alkyloxy include benzyloxy, naphthylmethyloxy, fluorenylmethyloxy, anthrylmethyloxy, biphenylylmethyloxy, tetrahydronaphthylmethyloxy, chromanylmethyloxy, 2,3-diliydro-1,4-diaxanapthalenylmethyloxy, indanylmethyloxy, phenanthrylmethyloxy, phenethyloxy, naphthylethyloxy, fluorenylethyloxy, anthrylethyloxy, biphenylylethyloxy, tetrahydronaphthylethyloxy, chromanylethyloxy, 2,3-diliydro-1,4-dioxanapthalenylethyloxy, indanylethyloxy, and phenanthrylethyloxy.

The compound represented by Formula (I) of the present invention has a structure in which amino acid, carboxylic acid or amine are linked in the order of (carboxylic acid)-(Ala)-(N-Me-Br-Tyr)-(Me-Pro)-(4-Me-Tzn)-(D-Leu)-(N-Me-Phe)-(2-Opp) from the N-terminal. Each component may be substituted with the following substituents.

Examples of derivatives of the compound represented by Formula (I) of the present invention are listed in (i) to (viii) below.

### (1) Carboxylic acid

Although Bisebromoamide (1) contains pivalic acid, the compound represented by Formula (I) of the present invention may contain aliphatic or aromatic carboxylic acid. These carboxylic acids may have one or more substituents such as hydroxy group, carbonyl group, ether group, halogen atom, cyano, nitro, amino, and carbamoyl.

### (i) Ala

The Ala residue may be substituted with Gly, Ser, Thr, β-Ala, or an N-alkylated (methylated, ethylated, propylated, butylated) amino acid residue thereof.

### (iii) N-Me-Br-Tyr

Br may be substituted with another substituent, or N-Me may be substituted with an alkyl group such as NH, N-Et, N-Pr, or N-Bu.

### (iv) Me-Pro

Regarding Me-Pro, alternations of configuration between D-isomer and L-isomer are included in the scope of the derivatives.

### (v) L-4-Me-Cys

Regarding L-4-Me-Cys, alternations of configuration between D-isomer and L-isomer are included in the scope of the derivatives.

### (vi) D-Leu

D, L, or DL-isomers of Leu, Ile, Val, norleucine, and norvaline can be used as the derivatives.

### (vii) N-Me-Phe

Phe, Tyr, phenylglycine, or an amino acid (Phe, Tyr) in which the amino group is substituted with methyl, ethyl, propyl, or butyl can be used; they may be either D-isomer or L-isomer.

### (viii) 2-Opp

Any tertiary amino groups can be used; and dialkylamino group, alkylarylamino group and like di-substituted amino groups, or piperidino, pyrrolidinyl, piperazinyl and like nitrogen-containing heterocyclic groups can be used. These may be substituted with a substituent such as R⁷-CO- or a hydrazide thereof, or R⁷-CH(OR⁸)- (wherein R⁷ represents an alkyl group, an aryl group, or an aralkyl group; and R⁸ represents a hydrogen atom, an alkyl group, an aryl group, an aralkyl group, or an acyl group).

The compound of the present invention contains asymmetric carbon atoms, and the configuration of each asymmetric carbon atom (R-configuration/S-configuration, or L-configuration/D-configuration) may be either one or a mixture thereof (including a racemic body). For example, when the compound represented by Formula (I) includes D-Leu, a compound in which the D-Leu is substituted with L-Leu is encompassed in the compound represented by Formula (I) of the present invention.

A salt of the compound represented by Formula (I) refers to that containing an acid addition salt or a basic salt in the structure. Specific examples of acid addition salts include inorganic salts such as hydrochloride, hydrobromide, hydroiodide, sulphate, perchlorate, and phosphate; organic salts such as oxalate, malonate, succinate, maleate, fumarate, lactate, malate, citrate, tartrate, benzoate, trifluoroacetate, acetate, methanesulfonate, p-toluenesulfonate, and trifluoromethanesulfonate; and acidic amino acid salts such as glutamate, and aspartate. Examples of basic salts include alkali metal salts or alkaline earth metal salts such as sodium salts, potassium salts, and calcium salts; salts with organic bases such as pyridine salts and triethylamine salts; and salts with basic amino acids such as lysine and arginine.

The compound of the present invention may be isolated from cyanobacterium collected in Okinawa, or produced by synthesis.

The synthesis scheme of the bisebromoamide represented by Formula (1) is shown below.

### Scheme 1

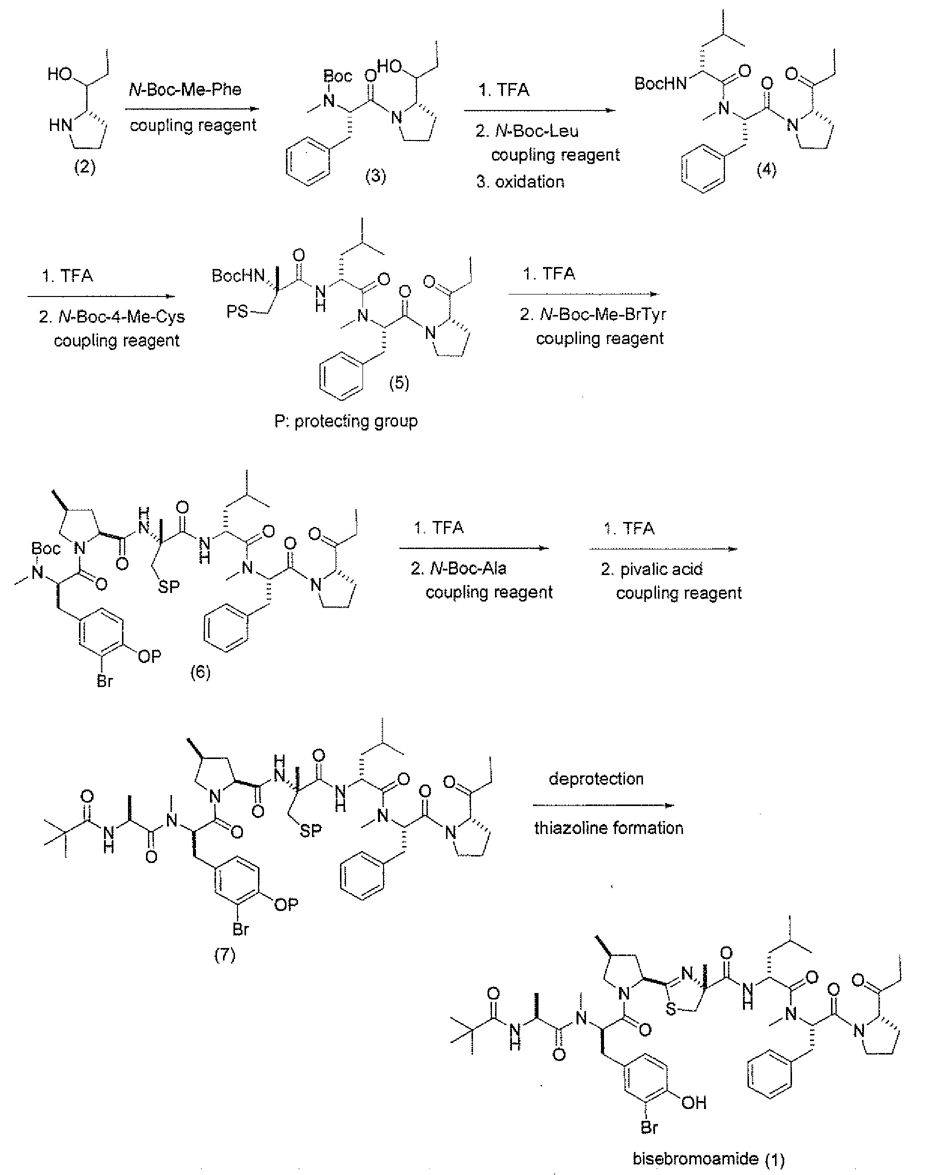

About 1 to 2 mol of N-Boc-Me-Phe is reacted per mole of 2-(1-hydroxypropyl)pyrrolidine (2) under the presence of a coupling reagent (dicyclohexylcarbodiimide (DCC), water-soluble carbodiimide, etc., 1 mol to an excess amount), leading to Compound (3). Subsequently, Boc group of Compound (3) (1 mol) is subjected to deprotection using TFA (1 mol to an excess amount) and allowed to react under the presence of N-Boc-D-Leu (about 1 to 2 mol) and a coupling reagent (DCC, water-soluble carbodiimide, etc., 1 mol to an excess amount), leading to Compound (4).
Thereafter, the reaction of subjecting 1 mol of Boc-protected amino acid to deprotection using TFA (1 mol to an excess amount), and condensing the resulting deprotected amino acid (1 mol) under the presence of a coupling reagent (DCC, water-soluble carbodiimide, etc., 1 mol to an excess amount) is performed repeatedly. After N-Boc-Ala, pivalic acid is reacted thereto, and the thiazoline ring is closed to obtain the target bisebromoamide of Formula (1). The reaction temperature, reaction time, and molar ratios of each reagent may be those generally used in peptide synthesis. For example, the reaction temperature is about 10 to 40°C, and the reaction time is about 30 min to 24 hours.
The amount of the reagent is, for example, when TFA (trifluoroacetic acid) is used, the amount thereof is 1 equivalent to an excess amount, and when a coupling reagent is used, the amount thereof is 1 mol to an excess amount per mole of raw material. The reaction favorably proceeds by conducting in an appropriate solvent such as ethyl acetate, THF, dioxane, DMF, and DMSO.

A compound represented by Formula (I) other than bisebromoamide or a derivative thereof can be produced by using, instead of (viii) Compound (2), (vii) N-Boc-Me-Phe, (vi) N-Boc-D-Leu, (v) N-Boc-4-Me-L-Cys, (iv) N-Boc-MePro, (iii) N-Boc-Me-BrTyr, (ii) N-Boc-Ala, and (i) pivalic acid, the aforesaid various compounds used for producing the compound represented by Formula (I) or the derivatives thereof.

The compound of the present invention is usually used as an anticancer drug in the form of a pharmaceutical composition prepared by mixing with a pharmaceutical carrier. Examples of pharmaceutical compositions include tablets, capsules, granules, powders, syrups, injections, patches, and suppositories. These pharmaceutical compositions can be prepared by an ordinary known method.

As the pharmaceutical carrier, a material that is conventionally used in the medicinal field and that does not react with the compound of the present invention is used. Specific examples of pharmaceutical carriers used for tablets, capsules, granules, and powders include excipients such as lactose, corn starch, saccharose, mannitol, calcium sulfate, and crystalline cellulose; disintegrants such as carmellose calcium, modified starch, and carmellose calcium; binders such as methylcellulose, gelatin, gum arabic, ethylcellulose, hydroxypropylcellulose, and polyvinylpyrrolidone; and lubricants such as light anhydrous silicic acid, magnesium stearate, talc, and hardened oil. Tablets may be coated by a known method using a coating agent such as carnauba wax, hydroxypropyl methylcellulose, macrogol, hydroxypropyl methylphthalate, cellulose acetate phthalate, saccharose, titanium oxide, sorbitan fatty acid ester, and calcium phosphate.

Examples of carriers used in the production of syrups include sweeteners such as saccharose, glucose, and fructose; suspending agents such as gum arabic, tragacanth, carmellose sodium, methylcellulose, sodium alginate, crystalline cellulose, and veegum; and dispersants such as sorbitan fatty acid ester, sodium lauryl sulfate, and Polysorbate 80. In the production of syrups, corrigents, flavoring agents, preservatives and the like may be added, if necessary. The syrups may be in the form of a dry syrup that is dissolved or suspended during use.

Examples of bases for suppositories include cacao butter, saturated fatty acid glycerol esters, glycerogelatin, and macrogol. In the preparation of suppositories, surfactants, preservatives, and the like may be used, if necessary.

These pharmaceutical compositions may usually contain, as an active ingredient, the compound represented by Formula (I), a derivative thereof, or a salt thereof in a proportion of 0.5% or more, and preferably 10 to 70%. These pharmaceutical compositions may further contain other therapeutically effective substances mentioned below.

The cancers or malignant tumors that can be treated by administering the compound of the present invention are not particularly limited, as long as the compound of the present invention exhibits sensibility thereto. Specific examples thereof include head and neck cancers, gastric cancers, colorectal cancers (colon cancers), liver cancers, gallbladder/bile duct cancers, pancreatic cancers, lung cancers, breast cancers, bladder cancers, prostatic cancers, cervical cancers, endometrial cancers, pharyngeal cancers, esophageal cancers, renal cancers, and ovarian cancers. The compound of the present invention can be an effective anticancer drug against cancers exhibiting accelerated activation in ERK pathway because it can selectively inhibit the MAPK signaling pathways, in particular, phosphorylation of ERK.

A pharmaceutical composition comprising the compound of the present invention can be used in combination with various medicines used for treating cancers depending on the conditions and the like of the patients. Examples of such medicines include irinotecan, cisplatin, carboplatin, oxaliplatin, 5-FU, dacarbazine, procarbazine, vinblastine, vincristine, taxol, docetaxel, gemcitabine, Glivec, Iressa, Tarceva and Nexavar, Herceptin, bevacizumab, cetuximab, nimotuzumab, sorafenib, and sunitinib.

### Examples

The present invention is described below in further detail with reference to Examples.

The structure determination and biological activity of the compound of the present invention are explained below.

### Example 1

### 1. Isolation of Bisebromoamide and Absolute Stereostructure thereof

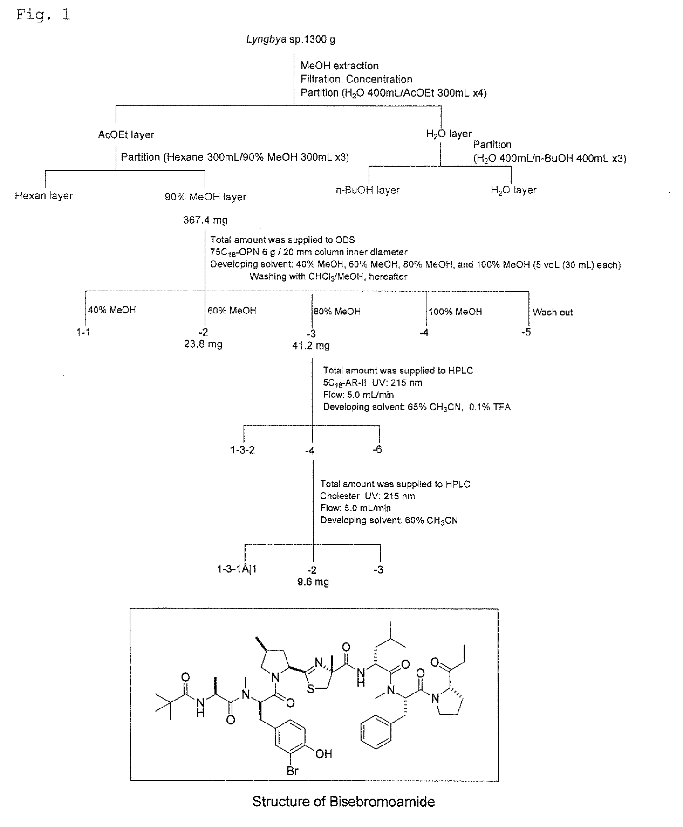

The cyanobacterium *Lyngbya* sp. collected in Okinawa Prefecture was extracted with MeOH, and partitioned between H₂O and AcOEt. The resulting AcOEt layer was further partitioned between 90% MeOH and n-hexane. The 90% MeOH layer thus obtained was separated and purified with various chromatographies using the growth inhibitory activity against tumor cells as an index. As a result, Bisebromoamide (1), which is a novel peptide, was isolated. The scheme until the isolation is shown in Fig. 1.

This compound exhibited strong growth inhibitory activity (IC₅₀40 ng/ml) against HeLa S₃ cell. The planar structure of the compound was primarily determined by various two-dimensional NMR spectral analyses. The results revealed that Bisebromoamide (1) contained many unusual amino acid structures; and comprised, as structural elements, Ala, N-Me-3-bromo-Tyr, 4-Me-Pro, 2-Me-Cys, Leu, N-Me-Phe, pivalic acid, and 2-(1-oxypropyl)pyrrolidine (Opp). Subsequently, in order to determine the absolute configuration of Bisebromoamide (1) including eight chiral centers, acid hydrolysis was conducted using 9 M HCl. The resulting acid hydrolysate was fractionated by HPLC, obtaining fractions of Leu, N-Me-Tyr, 4-Me-Pro, N-Me-Phe, Opp, and that of a mixture of Ala and 2-methylcystine. These fragments were analyzed by chiral HPLC, and the results revealed that the configurations of Leu, N-Me-Tyr, and N-Me-Phe were D, D, and L, respectively. Furthermore, 4-Me-Pro, Opp, Ala, and 2-methylcystine were subjected to derivatization using Marfey's reagent, and then analyzed by reversed phase HPLC. The results revealed that the configurations of Ala and 2-methylcystine were L and L, respectively. However, it also turned out that the Opp and 4-Me-Pro completely epimerized at a-position during the acid hydrolysis. Therefore, we reduced the ketone of Opp using NaBH₄ before conducting the acid hydrolysis. This prevented racemization at 2-position, and gave 2(S)-(1-hydrnxypropyl)-piperidine [6S : 6R= 1:1]. It became clear that the absolute configuration at the 2-position was S. Furthermore, 4-Me-Pro was subjected to acid hydrolysis after conducting ozonolysis to open the thiazoline ring. A comparison of the ¹H NMR data between the acid hydrolysate and authentic sample, and the HPLC analysis gave (4S)-4-Me-Pro [2S : 2R= 7:3]. It became clear that the absolute configuration of 4-Me-Pro was (2S, 4S).

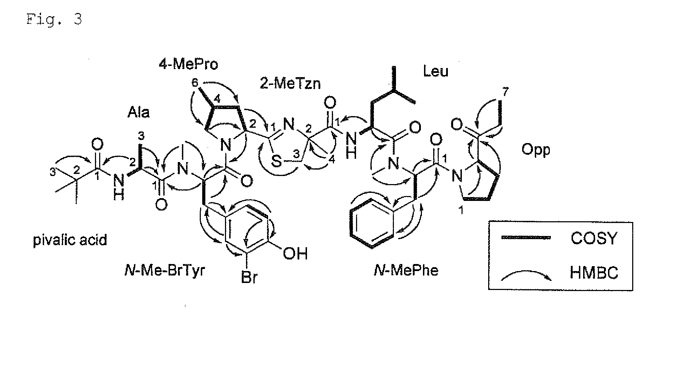

The stereostructure of Bisebromoamide (1) was determined primarily based on NMR spectral analysis. A detailed analysis of the COSY spectrum using chloroform-d revealed that two amide protons are individually linked to Alanine (Ala) and Leucine (Leu) residues. Further, a detailed analysis of COSY, HMQC, and HMBC using methanol-d revealed the presence of N-methyl-3-bromotyrosine (N-Me-BrTyr), 4-methylproline (4-MePro), N-methylphenylalanine (N-MePhe), and 2-(1-oxo-propyl)-pyrrolidine (Opp). In addition to these results, in HMBC data, a correlation from a methyl singlet (H-4; MeCys) to carbons C-1 (MeCys), C-2 (MeCys), and C-3 (MeCys) was observed, and a correlation from H-3 (MeCys) to C-1 (MePro) was also observed. This suggested the presence of a 2-methylthiazoline-2-carboxylic acid unit. Further detailed analysis of the HMBC spectrum revealed the linkage of six amino acid residues (Fig. 3). Furthermore, from the correlations between H3 (pivalic acid)/C1 (pivalic acid) and H2 (Ala)/C1 (pivalic acid), it became clear that there was a linkage between C1 (pivalic acid) and C2 (Ala), and Bisebromoamide (1) possessed an N-pivalamide moiety. Although no additional linkages were obtained from the NMR analysis, the linkage between Opp and N-MePhe became clear based on the molecular formula and degree of unsaturation. Thus, the structure of Bisebromoamide (1) was determined as shown in Fig. 3. The NMR data of Bisebromoamide are shown in Table 1 below.

**Table 1**

| Position | ¹H (ppm)^{a} | ¹³C (ppm)^{b} | Position | ¹H | (ppm)^{a} | ¹³C (ppm)^{b} |
|---|---|---|---|---|---|---|
| 2-Opp 1 | 3.54 m | 48.7 | 4-MePro | 1 | | 180.3 |
| 2 | 1.88 m | 25.7 | | 2 | 4.83 dd (10.7, 7.8) | 66.5 |
| 3 | 1.82 m, 2.20 m | 29.1 | | 3 | 1.67 m, 2.37 m | 39.6 |
| 4 | 4.56 dd (8.8, 4.4) | 62.6 | | 4 | 2.25 m | 35.1 |
| 5 | | 211.6 | | 5 | 2.94 m, 3.56 m | 55.7 |
| 6 | 2.54 q (7.3) | 33.6 | | 6 | 1.04 d (6.8) | 16.7 |
| 7 | 1.03 t (7.3) | 7.8 | *N*-Me-BrTyr | 1 | | 171.4 |
| *N*-MePhe 1 | | 170.4 | | 2 | 5.58 dd (9.3, 6.4) | 57.8 |
| 2 | 5.82 dd (10.3, 5.4) | 56.5 | | 3 | 2.96 m | 34.5 |
| 3 | 3.06 m | 35.7 | | 4 | | 131.2 |
| 4 | | 138.3 | | 5 | 7.36 d (2.0) | 135.1 |
| 5,9 | 7.27 dud (7.3, 2.4) | 130.7 | | 6 | | 110.3 |
| 6,8 | 7.20 dd (7.8, 7.3) | 129.4 | | 7 | | 154.1 |
| 7 | 7.09 dd (7.8, 2.4) | 127.7 | | 8 | 6.81 d (8.3) | 117.1 |
| 8 | 3.07 s | 31.1 | | 9 | 7.07 d (8.3, 2.0) | 131.0 |
| Leu 1 | | 174.3 | | 10 | 3.12 s | 32.3 |
| 2 | 4.52 dd (10.8, 3.4) | 50.2 | Ala | 1 | | 175.2 |
| 3 | 0.67 m, 1.57 m | 40.0 | | 2 | 4.65 m | 39.3 |
| 4 | 1,52 m | 25.7 | | 3 | 0.95 d (7.3) | 27.7 |
| 5 | 0.81 d (6.4) | 21.7 | Pivalic acid | 1 | | 180.7 |
| 6 | 0.79 d (6.4) | 23.7 | | 2 | | 39.3 |
| 2-MeTzn 1 | | 175.7 | | 3 | 1.14 s | 27.7 |
| 2 | | 84.9 | | | | |
| 3 | 3.23 d (11.2) | 43.5 | | | | |
| | 3.37 d (11.2) | | | | | |
| 4 | 1.52 s | 24.9 | | | | |

| | | | | | | |
|---|---|---|---|---|---|---|
| ^{a}Recorded at 400 MHz in CD₃OD. Coupling constants (Hz) are in parentheses. ^{b}Recorded at 100 MHz in CD₃OD. | | | | | | |

High Resolution Mass Spectrometry of Bisebromoamide (1)
HRMS (ESI) m/z 1044.4212,
calcd for C₅₁H₇₂⁷⁹BrN₇O₈SNa (M + Na)⁺ 1044.4244
Based on the results described above, the absolute stereostructure of Bisebromoamide (1) was determined (refer to Scheme 2).

### Scheme 2

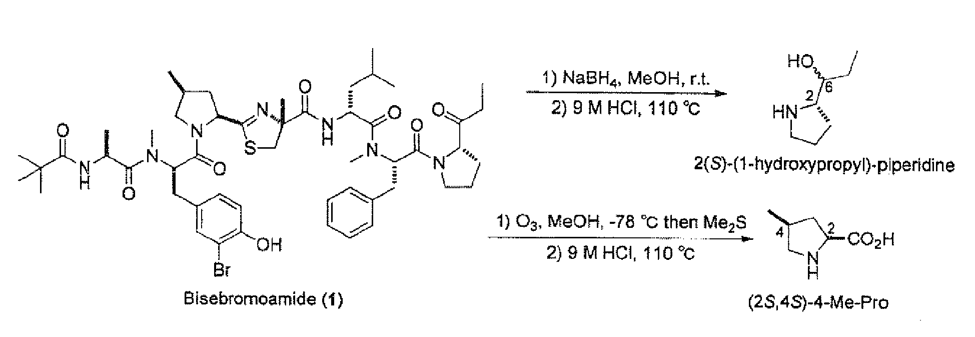

Bisebromoamide (1) is a strong cytotoxic substance containing a high degree of D-amino acids and unusual amino acids. In particular, compounds having a 4-Me-Pro, N-Me-3-bromo-Tyr, and N-pivalamide moiety linking to 4-methylthiazoline are very rare, and the 2-Opp unit is unprecedented in natural products. Bisebromoamide (1) was subjected to screening using 39 types of human cancer cells. Bisebromoamide (1) exhibited a strong growth inhibitory activity against various cancer cells. The average GI₅₀ value across all of the cell lines tested was 40 nM.

The growth inhibitory activity of Bisebromoamide (1) against each cancer cell line was tested as below.

### Measurement for Growth Inhibitory Activity Against Each Cancer Cell Line

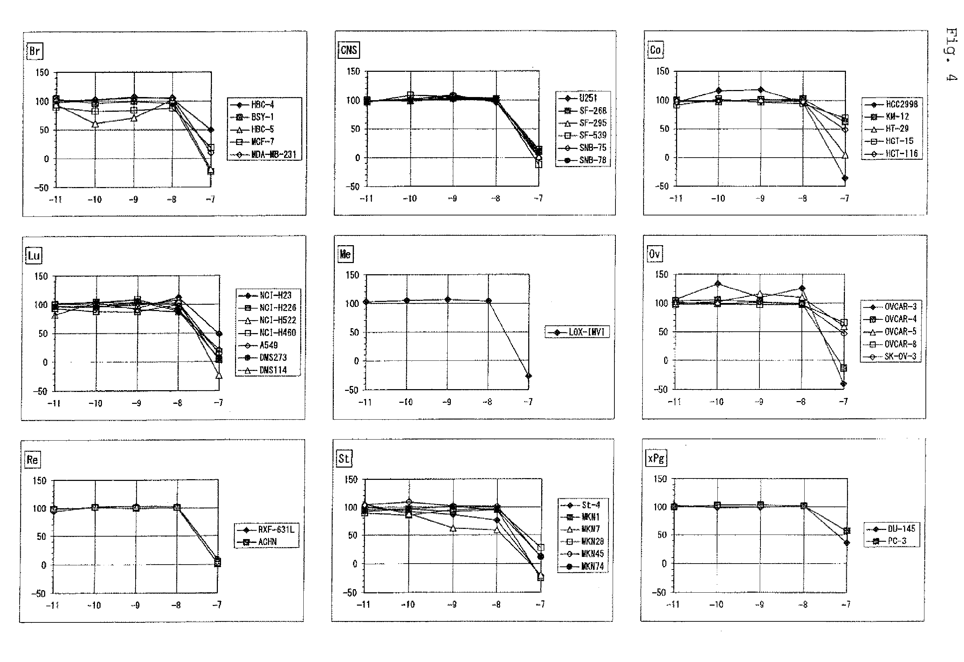

Cancer cells were seeded in a 96-well plate. A sample solution was added thereto on the following day, and cultured for 2 days. Cell growth was evaluated by sulforhodamine B colorimetric assay. The 39 total cancer cell lines that were tested included 7 lung cancer (Lu) lines, 6 stomach cancer (St) lines, 5 colon cancer (Co) lines, 6 brain neoplasm (CNS) lines, 5 breast cancer (Br) lines, 2 renal cancer (Re) lines, 2 prostatic cancer (xPg) lines, and 1 melanoma (Me) line. The effective concentration deviation of each cancer cell stock from the mean effective drug concentration of the 39 cancer cell lines measured was calculated and expressed as the fingerprint. Fig. 4 shows the results.

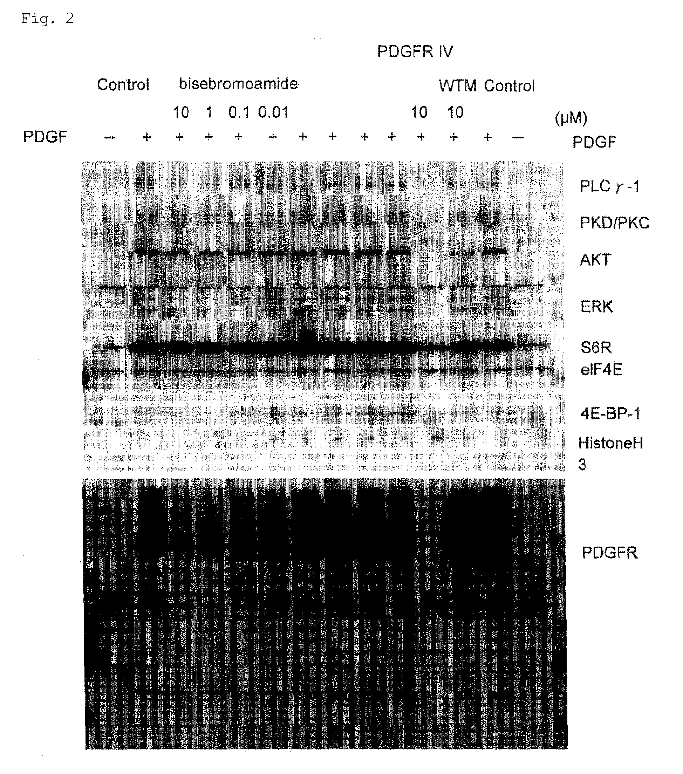

Bisebromoamide (1) exhibited a very strong protein kinase inhibitory activity. Bisebromoamide (1) selectively inhibited, in particular, the phosphorylation of ERK at 10, 1, and 0.1 µM; and it had no effect on the phosphorylation of AKT, PKD/PKC, PLCγ1 and S6 ribosomal protein (S6R) (Fig. 2). The method for measuring the protein kinase inhibition is explained below.

### Method of Measuring Protein Kinase Inhibition

### Method

NRK cells were seeded in a 96-well plate, and cultured for 3 days. A sample solution was added thereto and treated for 3 hours, followed by PDGF stimulation. Electrophoretic samples were prepared to conduct Western blot using antibodies against phosphorylated signal transduction molecules (AKT, ERK, PKD, PLCγ1, and S6 ribosomal protein) and phosphotyrosine. The effect on the intracellular signal transduction from a PDGF receptor was evaluated. The same experiment was conducted for PDGFR inhibitor (PDGFR IV) and wortmannin (WTM) (because PDGFR IV inhibits the self-phosphorylation of PDGF receptor, the phosphorylation as a whole is lowered). The eIF4E, 4E-EP-1, and histone H3 bands confirmed that equal amounts of proteins subjected to electrophoresis. Fig. 2 shows the results.

### Evaluation of Activity

Bisebromoamide (1) was added in such a manner that the final concentration became 0.01, 0.1, 1, and 10 µM, in order to determine the presence or absence of inhibition, and the inhibition pattern thereof. The effective inhibitory concentration against each signal transduction molecule activity was investigated.

### Example 2: Production Example for obtaining a hydrazone of Opp in Bisebromoamide (1) and an alcohol by reducing the ketone of Opp in Bisebromoamide (1)

### Reduction of Bisebromoamide (alcohol)

Sodium borohydride (30 mg, 0.79 mmol) was added to a 0.5 mL of methanol solution of Bisebromoamide (2.5 mg, 2.44 µmol). The reaction system was stirred at room temperature for 2 hours, diluted with 5 mL of water and 5 mL of ethyl acetate, and then extracted with 10 mL of ethyl acetate 3 times. The organic layer was washed with a 15 mL of saturated sodium chloride solution, dried with anhydrous sodium sulfate, and then concentrated. The resulting concentrate was purified by silica gel column chromatography (5 g, hexane:ethyl acetate = 1:15) to give alcohol as a colorless oil (2.1 mg, 84%).
¹H NMR (270 MHz, CDCl₃)d 7.42 (d, J = 6.8 Hz, 1H), 7.31 (d, J= 2.0 Hz, 1H), 7.20 (d, J = 7.3, 1.5 Hz, 2H), 7.13 (dd, J = 7.8, 7.3 Hz, 2H), 7.09 (dd, J = 7.8, 1.5 Hz, 1H), 7.07 (d, J = 8.3, 2.0 Hz, 1H), 6.84 (d, J = 8.3 Hz, 1H), 6.28 (d, J = 7.8 Hz, 1H), 5.83 (dd, J = 11.2, 5.9 Hz, 1H), 5.59 (dd, J = 9.3, 7.8 Hz, 1H), 4.87 (dd, J = 10.7, 7.3 Hz, 1H), 4.70 (m, 1H), 4.50 (m, 1H), 4.12 (m, 1H), 3.60 (m, 2H), 3.52 (m, 1H), 3.39 (d, J = 11.2 Hz, 1H), 3.10 (d, J = 11.2 Hz, 1H), 3.08 (s, 3H), 3.06 (s, 3H), 3.06 (m, 2H), 3.05 (m, 2H), 2.89 (m, 1H), 2.35 (m, 1H), 2.19 (m, 1H), 2.11 (m, 1H), 1.88 (m, 2H), 1.80 (m, 1H), 1.74 (m, 1H), 1.55 (s, 3H), 1.50 (m, 2H), 1.43 (m, 1H), 1.41 (m, 1H), 1.17 (s, 9H), 1.03 (d, J = 5.9 Hz, 3H), 0.90 (t, J= 7.3 Hz, 3H), 0.87 (d, J = 7.3 Hz, 3H), 0.76 (d, J = 6.8 Hz, 3H), 0.74 (d, J = 6.8 Hz, 3H), 0.54 (m, 1H).

### Hydrazone of Bisebromoamide

0.3 mL of concentrated sulfuric acid was added to a 5 mL of methanol solution of 2,4-dinitrophenylhydrazine (125 mg, 0.63 mmol). 3 mL of the resulting solution was added to a 2 mL of methanol solution of bisebromoamide (3.3 mg, 3.22 mmol) dropwise and stirred at room temperature for 3 hours. The reaction system was diluted with 5 mL of water and 5 mL of ethyl acetate, and then extracted with 15 mL of ethyl acetate 3 times. The organic layer was washed with a 15 mL of saturated sodium chloride solution, dried with anhydrous sodium sulfate, and then concentrated. The resulting concentrate was purified by PLC (hexane:ethyl acetate = 1:15) to give a hydrazone as a yellow oil (3.6 mg, 92%). TLC, R_{f} 0.58 (hexane:ethyl acetate 1:15). ¹H NMR (270 MHz, CDCl₃) d 7.42 (d, J = 6.8 Hz, 1H), 7.31 (d, J = 2.0 Hz, 1H), 7.20 (d, J = 7.3, 1.5 Hz, 2H), 7.13 (dd, J = 7.8, 7.3 Hz, 2H), 7.09 (dd, J = 7.8, 1.5 Hz, 1H), 7.07 (d, J = 8.3, 2.0 Hz, 1H), 6.84 (d, J = 8.3 Hz, 1H), 6.28 (d, J = 7.8 Hz, 1H), 5.83 (dd, J = 11.2, 5.9 Hz, 1H), 5.59 (dd, J = 9.3, 7.8 Hz, 1H), 4.87 (dd, J = 10.7, 7.3 Hz, 1H), 4.70 (m, 1H), 4.50 (m, 1H), 4.12 (m, 1H), 3.60 (m, 2H), 3.52 (m, 1H), 3.39 (d, J = 11.2 Hz, 1H), 3.10 (d, J = 11.2 Hz, 1H), 3.08 (s, 3H), 3.06 (s, 3H), 3.06 (m, 2H), 3.05 (m, 2H), 2.89 (m, 1H), 2.35 (m, 1H), 2.19 (m, 1H), 2.11 (m, 1H), 1.88 (m, 2H), 1.80 (m, 1H), 1.74 (m, 1H), 1.55 (s, 3H), 1.50 (m, 2H), 1.43 (m, 1H), 1.41 (m, 1H), 1.17 (s, 9H), 1.03 (d, J = 5.9 Hz, 3H), 0.90 (t, J= 7.3 Hz, 3H), 0.87 (d, J = 7.3 Hz, 3H), 0.76 (d, J = 6.8 Hz, 3H), 0.74 (d, J = 6.8 Hz, 3H), 0.54 (m, 1H).
The alcohol and hydrazone were examined for their inhibitory activities against cancer cell growth and phosphorylation of ERK. Results similar to those for Bisebromoamide (1) were obtained.

## Claims

1. A compound represented by Formula (I) below, a derivative thereof, or a salt thereof: 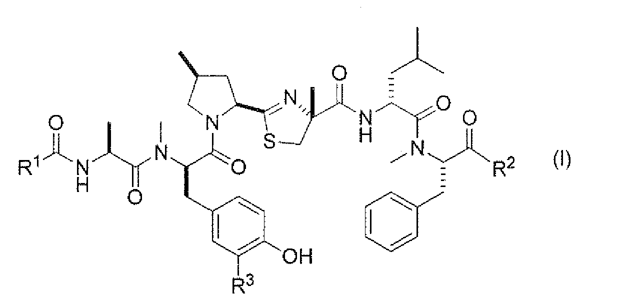 wherein R¹ represents an alkyl group, an alkenyl group, a cycloalkyl group, an amino group, a monoalkylamino group, a dialkylamino group, an aryl group, a heteroaryl group, an aralkyl group or an alkoxy group;
R² represents a substituted or unsubstituted amino group, or a substituted or unsubstituted nitrogen-containing heterocyclic group;
R³ represents hydrogen, halogen, alkyl, methoxy, cyano, trifluoromethyl, acetylamino or amino;
provided that Ala in Formula (I) may be substituted with N-Me-Gly, N-Me-Ser, N-Me-Thr, N-Me-Ala, Gly, Ser or Thr;
N-Me-Phe may be substituted with Phe, Tyr or N-Me-Tyr; and
D-Leu may be substituted with L-Leu.

2. The compound of claim 1, a derivative thereof, or a salt thereof, wherein R² represents NR⁴R⁵ (wherein R⁴ and R⁵ are the same or different, and each represents a hydrogen atom, an alkyl group, or a cycloalkyl group), 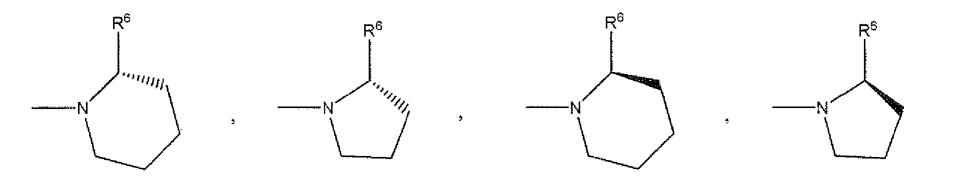 wherein R⁶ represents R⁷-CO- or a hydrazide thereof, or R⁷-CH(OR⁸)-(wherein R⁷ represents an alkyl group, an aryl group, or an aralkyl group; and R⁸ represents a hydrogen atom, an alkyl group, an aryl group, an aralkyl group, or an acyl group).

3. The compound of claim 1 represented by Formula 1 below, a derivative thereof, or a salt thereof:

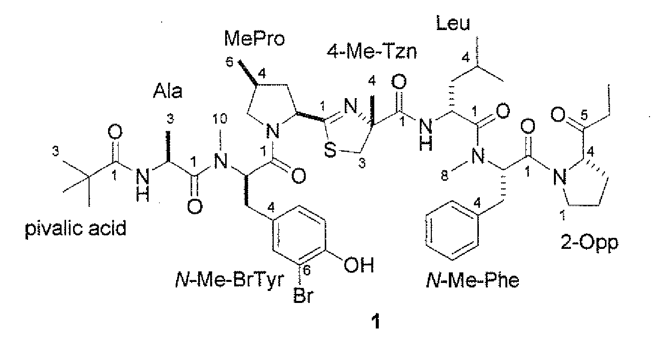

4. An anticancer drug comprising the compound of any one of claims 1 to 3, a derivative thereof, or a salt thereof as an active ingredient.

5. An ERK-selective phosphorylation inhibitor comprising the compound of any one of Items 1 to 3, a derivative thereof, or a salt thereof as an active ingredient.
